# EUROPEAN PATENT APPLICATION

(11) **EP 1 982 647 A2**
(43) Date of publication of application: **22.10.2008**
(21) Application number: 08251470.4
(22) Date of filing: 21.04.2008
(51) Int. Cl.: A61B 5/021, A61B 5/0402

(54) **Pressure measurement-based ischemia detection**

(30) Priority: 19.04.2007 US 737650
(71) Applicant: PACESETTER, INC., Sylmar, CA 91392-9221 (US)
(72) Inventor: Shelchuk Anne M., Cupertino, CA 95014 (US); Gutfinger, Dan E., Irvine, CA 92604 (US)
(74) Representative: Phillips, Emily Elizabeth

(57) **Abstract**

In some aspects ischemia is indicated bases on cardiac pressure measurements. For example, ischemia may be indicated based on an increase in an intraventricular electromechanical delay where mechanical contractions are detected by measuring pressure in a cardiac chamber. Ischemia detection also may involve obtaining timing information relating to a mechanical contraction of at least one ventricle to identify the timing of the mechanical contraction. Ischemia may be indicated based on a change in a time interval associated with a systolic interval of a ventricle. Ischemia also may be indicated based on an intracardiac electrogram-based indication of ischemia in conjunction with an increase in mean left atrial pressure and/or increase in the size of a v-wave.

## Description

This application is related to U.S. Patent Application Serial No. 11/537,302, filed September 29, 2006 entitled "Estimating Mean Left Atrial Pressure" (Atty. Docket No. A06P3023US01); and U.S. Patent Application Serial No. 11/537,622, filed September 29, 2006 entitled "Monitoring for Mitral Valve Regurgitation" (Atty. Docket No. A06P3023US02); and U.S. Patent Application Serial No. 11/557,887, filed November 6, 2006 entitled "Systems and Methods for Evaluating Ventricular Dyssynchrony Using Atrial and Ventricular Pressure Measurements Obtained by an Implantable Medical Device (Atty. Docket No. A06P1121).

This application relates generally to detection of cardiac ischemia, and to measuring pressure to generate an indication of ischemia.

Cardiac ischemia is a condition whereby heart tissue does not receive an adequate amount of oxygen. Ischemia may occur chronically, and to varying degrees, as a result of coronary artery disease or acutely as a result of sudden increased demand, embolism or vasospasm. Ischemia can lead to angina and eventually to myocardial infarction, i.e., permanent damage to the heart muscle. Both ischemia and infarction may potentially trigger fatal arrhythmias.

Ischemia has traditionally been detected by monitoring cardiac electrical signals as represented by a surface electrocardiogram ("surface ECG") or an intracardiac electrogram ("IEGM"). For example, a change in the level of the ST segment of a QRST complex of the surface ECG or IEGM is a known indicator of ischemia.

Surface ECGs are typically taken in a clinical setting using a relatively large number of leads. As a result, relatively accurate detection of ischemia may be made since the electrophysiological conditions of the patient may be closely monitored or controlled. However, this technique only detects ischemia events that occur when the patient is in the clinic.

IEGMs, on the other hand, may be recorded using an implantable cardiac device such as a pacemaker or an implantable cardioverter defibrillator ("ICD"). An implantable cardiac device may be configured to repeatedly monitor the electrophysiological conditions of a patient using a few implanted lead electrodes. Accordingly, more ischemia information may be obtained through the use of an implantable cardiac device.

In practice, however, some inaccuracies may result from an ischemia detection technique based on IEGM information. For example, since the IEGM information is not acquired in a clinical setting, a variety of environmental or patient-related factors other than ischemia may affect the IEGM parameters being monitored. In particular, changes in the ST segment also may be caused by changes in the activity level and/or the position of a patient. Moreover, the IEGM is generally collected using only a few implanted leads. Consequently, it may not be possible to obtain the same degree of specificity regarding the localization of an ischemia as may be obtained using an ECG-based approach.

According to the invention there is provided a system for of generating an indication of ischemia associated with a heart of a patient, comprising: delay determining means determining an electrical depolarization to mechanical contraction delay for a ventricle of a heart; means for determining whether the delay is more than an expected delay; and means for generating an indication of ischemia in accordance with a determination that the delay is more than the expected delay.

The invention also extends to a method of generating an indication of ischemia associated with a heart of a patient, comprising: determining an electrical depolarization to mechanical contraction delay for a ventricle of a heart; determining whether the delay is more than an expected delay; and generating an indication of ischemia in accordance with a determination that the delay is more than the expected delay.

**[0009a]** Ischemia may be indicated based on cardiac pressure measurements. For example, ischemia detection may involve monitoring one or more features derived from pressure readings obtained from a chamber of a patients heart. An increase or a decrease in such a feature may result in generation of an indication of ischemia. For example, electromechanical synchronicity information contained pressure signals associated with the ventricles (e.g., left atrial pressure signals and right ventricular pressure signals) may be used to detect ischemia or increase the specificity of ischemia detection.

Ischemia may be indicated based on an increase in an intraventricular electromechanical delay. For example, the delay between an electrical depolarization event and the subsequent mechanical contraction for a ventricle may be monitored over time. In the event this delay increases, ischemia may be indicated for that ventricle.

Mechanical contraction for a ventricle may be detected by measuring pressure associated with the ventricle. For example, in some embodiments a pressure sensor may be implanted in the ventricular chamber. In this case, an upslope of the pressure in the chamber may be detected to indicate the onset of the mechanical contraction of the ventricle. Alternatively, in some embodiments a pressure sensor may be implanted in an atrial chamber associated with the ventricular chamber. In this case, a c-wave in the atrial chamber may be detected to indicate the onset of the mechanical contraction of the ventricle.

Ischemia may be indicated based on identification of an interventricular dyssynchrony. Here, pressure measurements are used to identify the timing of a mechanical contraction of a ventricle. The timing of the mechanical contraction, in turn, is used to identify the interventricular dyssynchrony. In some embodiments the timing between the upslope of a right ventricle pressure signal and a c-wave in a left atrial pressure waveform is monitored to identify the interventricular dyssynchrony. In some embodiments, timing between an electrical depolarization event and mechanical contraction may be monitored to identify the interventricular dyssynchrony.

Ischemia may be indicated based on a change in a systolic-related interval of a ventricle (e.g., the time duration of mechanical contraction of the ventricle). For example, ischemia may be indicated when a systolic interval decreases or when a diastolic interval increases. In some embodiments the systolic interval is obtained by measuring the interval between the upstroke and the downstroke of a ventricular pressure signal. In some embodiments the systolic interval is obtained by measuring the interval between the c-wave and the v-wave of an atrial pressure signal.

An indication of ischemia may be based on a pressure-based detection of ischemia considered independently or in combination with some other form of ischemia detection. For example, a preliminary indication of ischemia based on pressure measurements may be used to corroborate a preliminary indication of ischemia that is generated based on IEGM information (e.g., a change in the level of the ST segment).

Ischemia may be indicated based on an intracardiac electrogram-based indication of ischemia and an increase in mean left atrial pressure. For example, ventricular ischemia may reduce ventricular function which, in turn, may cause an increase in left atrial pressure. In some embodiments the increase in mean left atrial pressure is detected by determining whether there has been an increase in an absolute mean pressure or in a relative mean pressure associated with first and second time intervals.

Ischemia may be indicated based on an intracardiac electrogram-based indication of ischemia and an increase in the size (e.g., magnitude) of a v-wave. For example, ventricular ischemia will often result in mitral valve regurgitation as the papillary muscles become ischemic and impair valve function. Mitral valve regurgitation may appear as enlarged v-waves in the left atrial pressure signal. In some embodiments the increase in the size of the v-wave is detected by determining whether there has been an increase in an absolute v-wave pressure parameter or in a relative mean left atrial pressure associated with first and second time intervals. In some embodiments, the presence of mitral valve regurgitation alone, or in conjunction with an increase in mean left atrial pressure, may be used in concert with electrogram-based ischemia detection to increase the specificity of diagnosis.

Pressure-based ischemia detection may provide localization specificity. For example, a pressure measurement may indicate that an ischemia is associated with a given ventricle. Such localization specificity may be provided in conjunction with a pressure-based detection of ischemia considered independently or in combination with some other form of ischemia detection.

The techniques described herein may be implemented in one or more devices of various types. For example, in some embodiments these techniques may be implemented in an implantable device, a programmer, a patient handheld communicator or a remote, centralized patient monitoring system.

These and other features, aspects and advantages of the invention will be more fully understood when considered with respect to the following detailed description, appended claims and accompanying drawings, wherein:

FIG. 1 is a flowchart of an embodiment of operations that may be performed to generate an indication of ischemia;

FIG. 2 is a flowchart of an embodiment of operations that may be performed to repeatedly monitor for ischemia;

FIG. 3 is a flowchart of an embodiment of operations that may be performed to generate an indication of ischemia based on an increase in an electrical depolarization to mechanical contraction delay;

FIG. 4 is a simplified diagram illustrating an example of a ventricular pressure waveform;

FIG. 5 is a simplified diagram illustrating an example of an atrial pressure waveform and a ventricular pressure waveform;

FIG. 6 is a flowchart of an embodiment of operations that may be performed to generate an indication of ischemia based on interventricular dyssynchrony derived from pressure measurements;

FIG. 7 is a simplified diagram illustrating an example of an atrial pressure waveform and a ventricular pressure waveform;

FIG. 8 is a flowchart of an embodiment of operations that may be performed to generate an indication of ischemia based on interventricular dyssynchrony;

FIG. 9 is a flowchart of an embodiment of operations that may be performed to generate an indication of ischemia based on a change in a systolic-related interval;

FIG. 10 is a flowchart of an embodiment of operations that may be performed to generate an indication of ischemia based on mean left atrial pressure;

FIG. 11 is a simplified diagram illustrating an example of a left atrial pressure waveform;

FIG. 12 is a flowchart of an embodiment of operations that may be performed to generate an indication of ischemia based on an increase in the size of a v-wave;

FIG. 13 is a simplified diagram of an embodiment of an implantable stimulation device in electrical communication with at least three leads implanted in a patient's heart for providing multi-chamber sensing and stimulation therapy; and

FIG. 14 is a simplified functional block diagram of an embodiment of a multi-chamber implantable stimulation device, illustrating basic elements that may be configured to provide ischemia detection, cardioversion, defibrillation or pacing stimulation or any combination thereof.

In accordance with common practice the various features illustrated in the drawings may not be drawn to scale. Accordingly, the dimensions of the various features may be arbitrarily expanded or reduced for clarity. In addition, some of the drawings may be simplified for clarity. Thus, the drawings may not depict all of the components of a given apparatus or method. Finally, like reference numerals may be used to denote like features throughout the specification and figures.

### Detailed Description

The invention is described below, with reference to detailed illustrative embodiments. It will be apparent that the invention may be embodied in a wide variety of forms, some of which may appear to be quite different from those of the disclosed embodiments. Consequently, the specific structural and/or functional details disclosed herein are merely representative and do not limit the scope of the invention. For example, based on the teachings herein one skilled in the art should appreciate that the various structural and/or functional details disclosed herein may be incorporated in an embodiment independently of any other structural and/or functional details. Thus, an apparatus may be implemented and/or a method practiced using any number of the structural and/or functional details set forth in any disclosed embodiment(s). Also, an apparatus may be implemented and/or a method practiced using other structural and/or functional details in addition to or other than the structural and/or functional details set forth in any disclosed embodiment(s).

An indication of ischemia may be generated, at least in part, based on pressure measurements taken from one or more chambers of a patient's heart. Here, one or more parameters derived from these pressure measurements may be monitored for any changes indicative of ischemia.

A brief overview of several exemplary parameters follows. An interval between an R-wave (as detected, for example, by a right ventricle electrode) and an upstroke of a right ventricle pressure signal may be referred to as the intraventricular electromechanical delay for the right ventricle. An interval between the R-wave (as detected, for example, by a left ventricle/coronary sinus electrode) and a c-wave in a left atrial pressure waveform may be referred to as the intraventricular electromechanical delay for the left ventricle. A difference between the upstroke of the right ventricle pressure signal and the c-wave in the left atrial pressure waveform may be referred to as an interventricular dyssynchrony. An interval between the c-wave and a v-wave in the left atrial pressure waveform may be referred to as the left ventricle systolic interval. A difference between a left ventricle-based R-wave to R-wave interval and the c-wave to v-wave interval may be referred to as the left ventricle diastolic interval. An interval between the upstroke and the downstroke of the right ventricle pressure signal may be referred to as the right ventricle systolic interval. The right ventricle diastolic interval may be defined as the balance between this and the right-side R-wave to R-wave interval.

In general, ventricular ischemia compromises ventricular mechanical function. An increase in the intraventricular electromechanical delay for the right ventricle may be indicative of a right-sided ischemic event, in particular when considered in conjunction with electrogram-based ischemia detection. An increase in the intraventricular electromechanical delay for the left ventricle may be indicative of a left-sided ischemic event, in particular when considered in conjunction with electrogram-based ischemia detection. A decrease in the systolic interval of either the right ventricle or the left ventricle may indicate an ischemic event in that portion of the heart.

In view of these and other relationships, several techniques for detecting ischemia are described herein. For example, in some embodiments ischemia detection may involve identifying an increase in an intraventricular electromechanical delay where mechanical contractions are detected via pressure measurements. In some embodiments ischemia detection also may involve obtaining timing information relating to a mechanical contraction of at least one ventricle to identify an interventricular dyssynchrony. Again, pressure measurements may be used to identify the timing of the mechanical contraction. In some embodiments ischemia may be indicated based on a change in a time interval associated with a systolic interval of a ventricle. Ischemia also may be indicated based on an increase in mean left atrial pressure and/or an increase in the size of a v-wave. These parameters and associated operations for obtaining and processing these parameters will be treated in more detail in the following description.

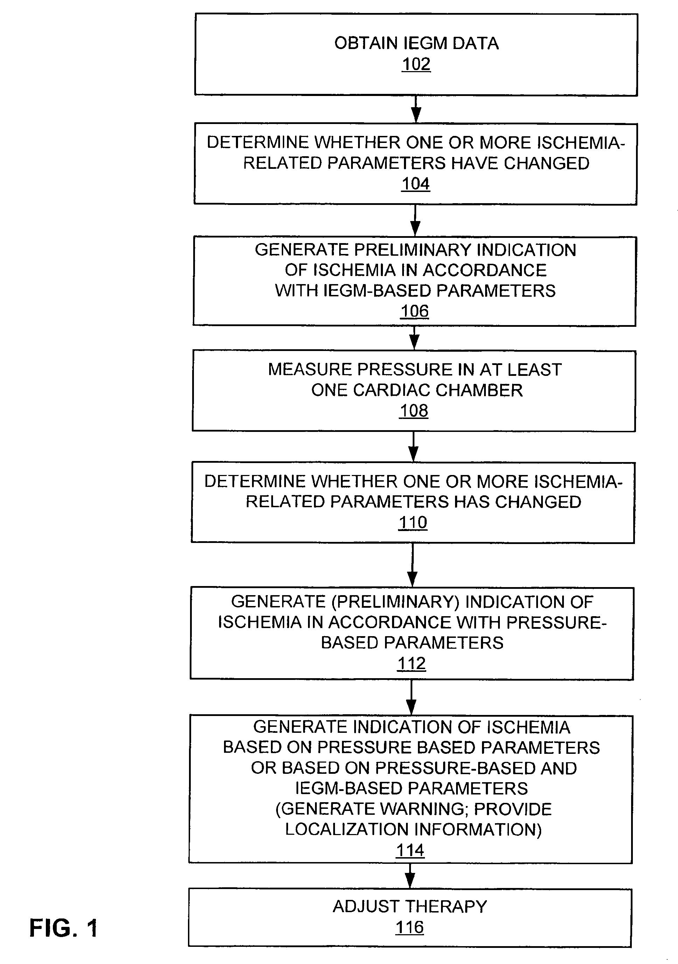

FIG. 1 illustrates an embodiment of basic operations that may be performed to generate an indication of ischemia. That is, these operations may be employed in conjunction with any of the specific parameter-based ischemia detection operations that are discussed in more detail later in this description. For convenience, the operations of FIG. 1 (and any other operations herein) may be described as being performed by specific components such as an implantable cardiac device (hereafter "the device"). It should be appreciated, however, that these operations may be performed in conjunction with or by other components.

The device may generate an indication of ischemia in some embodiments based solely on parameters derived from pressure measurements, or in other embodiments based on these parameters as well as an IEGM-based indication of ischemia. As an example of the latter embodiments, a pressure-based ischemia indication may be used to corroborate an IEGM-based ischemia indication. FIG. 1 illustrates operations that the device may perform in conjunction with either of these embodiments.

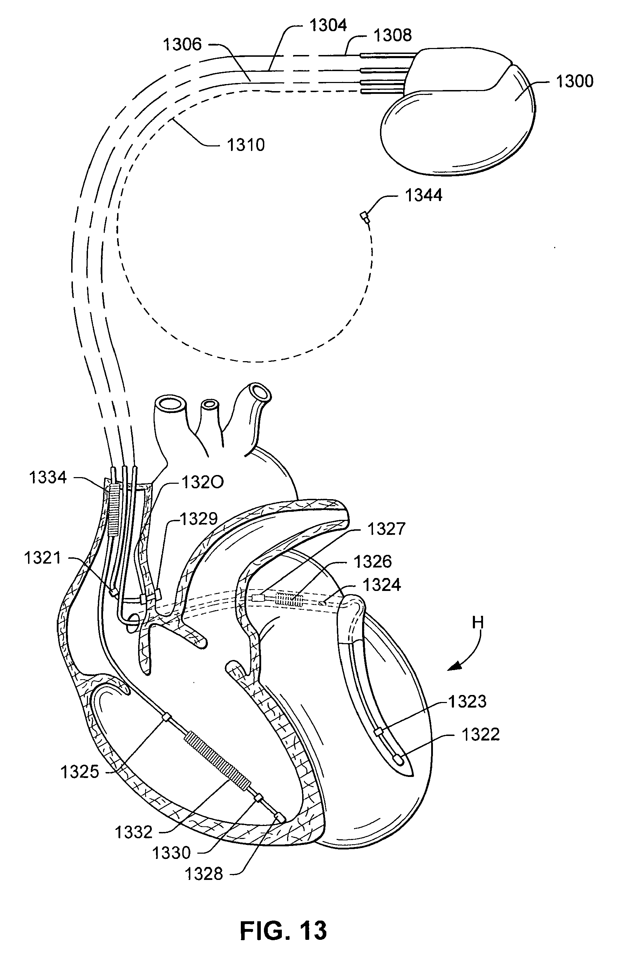

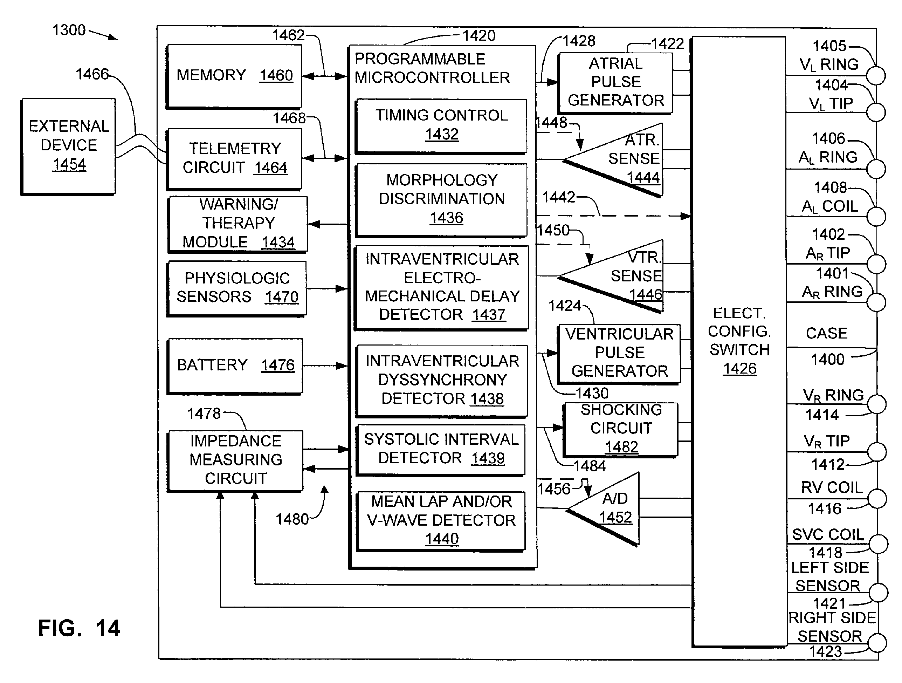

As represented by block 102, the device obtains IEGM data by, for example, repeatedly sensing cardiac activity over every cardiac cycle via one or more cardiac leads implanted in the heart of the patient. This operation will be discussed in more detail in conjunction with FIGS. 13 and 14. Of note here is that the IEGM includes several components indicative of electrical-based functions of the heart during a cardiac cycle. In particular, a P-wave corresponds to depolarization of an atrium, a QRS complex (which may be referred to as an R-wave) corresponds to depolarization of a corresponding ventricle and a T-wave corresponds to repolarization of the ventricle. Some of these parameters are used in embodiments that employ IEGM-based ischemia detection. In addition, some of these parameters may be used in conjunction with pressure-based parameters to generate an indication of ischemia.

Blocks 104 and 106 relate to operations that the device may perform in an embodiment that generates an indication of ischemia based, in part, on IEGM-based ischemia detection. Here, as represented by block 104, the device will regularly monitor one or more parameters of the IEGM data that may provide some indication of an ischemia. For example, the device may analyze the IEGM signal for a change in a level of the ST segment of the QRST complex and/or a change in a timing interval relating to the maximum amplitude point of a T-wave (referred to herein as "Tmax") of the QRST complex. Here, an increase in the level of the ST segment may indicate ischemia. In addition, a decrease in a time period Q-to-Tmax may indicate ischemia.

As represented by block 106, in the event the device determines that one or more ischemia-related parameters have changed, the device may generate an indication of ischemia. In practice, however, changes in an IEGM-based ischemia indicator such as the level of the ST segment may not always be due to ischemia. For example, the ST segment may elevate after a patient commences exercise. In addition, the ST segment may elevate when a patient moves from one body position (e.g., prone) to a different body position (e.g., upright). Accordingly, a final determination as to whether an ischemia is present may not be made from an IEGM-based ischemia indication alone. Rather, in some embodiments the IEGM-based ischemia indication may be designated as a preliminary indication to be corroborated by some other indication of ischemia.

Blocks 108, 110 and 112 relate to operations that may be performed to generate an indication of ischemia in accordance with pressure-based parameters. As represented by block 108, the device obtains pressure measurements from one or more chambers of the heart. As will be discussed in more detail in conjunction with FIGS. 13 and 14, this may be accomplished through the use of one or more pressure sensors implanted to sense pressure in a ventricular chamber and/or an atrial chamber.

As represented by block 110, the device will regularly monitor one or more pressure related parameters that may provide some indication of an ischemia. For example, as discussed above the device may monitor left atrial pressure and timing parameters related to a mechanical contraction and an atrial pressure waveform. As will be discussed in more detail below, certain changes in these parameters may indicate an ischemic condition.

As represented by block 112, in the event the device determines that one or more ischemia-related parameters have changed, the device may generate an indication of ischemia. As discussed above, in some embodiments the pressure-based ischemia indication may be used as an ultimate ischemia indicator.

Conversely, in some embodiments a pressure-based ischemia indication may be used as a preliminary indication that may be combined with some other ischemia indicator to generate an ultimate ischemia indication. For example, as represented by block 114 an ultimate ischemia indication may be generated based on corroboration of the preliminary indications from blocks 106 and 112. Here, it should be appreciated that FIG. 1 illustrates but one example order of operations. Thus, a pressure-based ischemia indication may be used (e.g., invoked) to substantiate an IEGM-based ischemia indication or vice versa. Alternatively, each indication may be invoked independently whereby the operations of block 114 are based on the results of these independent operations.

An ischemia indication may take various forms. In some embodiments this may involve one or more of setting a corresponding parameter, generating a warning signal (e.g., a vibratory, audible or tickler signal) or sending an indication to an external device (e.g., via a radio frequency signal). In the latter case, the external device may send a notification to the patient, the patient's physician or some other person or entity via an appropriate communication mechanism (e.g., a telephone system or a data network).

As represented by block 116, in some embodiments therapy may be administered to the patient based on the ischemia indication. For example, therapy (e.g., a drug) may be administered to the patient or a prescribed therapy may be modified. To this end, the implanted apparatus may include a drug delivery mechanism or the implanted apparatus may generate an indication (e.g., a signal) that causes a separate drug delivery mechanism to deliver a drug. In some embodiments the apparatus may adjust cardiac pacing timing in response to an indication of ischemia.

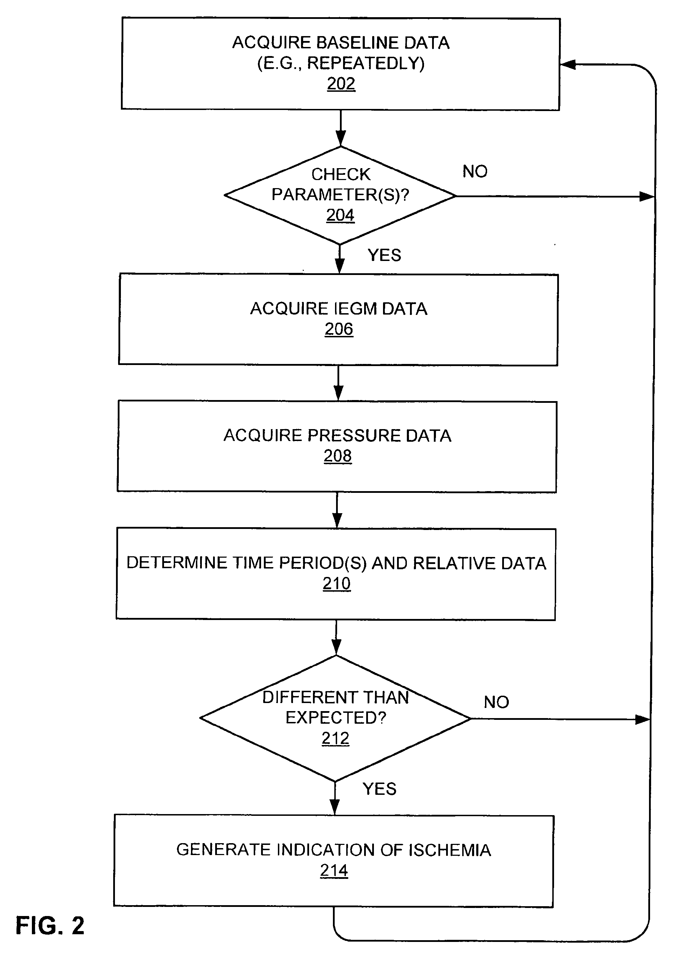

FIG. 2 illustrates an embodiment of basic operations that may be repeatedly performed to collect ischemia-related parameters and determine whether any change in one or more of these parameters is indicative of ischemia. As in FIG. 1, these operations may be employed in conjunction with any of the specific parameter-based ischemia detection operations that are discussed in more detail later in this description.

As represented by block 202, in some embodiments an initial operation involves obtaining baseline data from the patient. The device may use the baseline data in subsequent operations to identify any changes in ischemia-related parameters. Thus, the baseline data may represent the patient's condition in the absence of ischemia. As will be discussed in more detail below, the baseline data may represent, without limitation, timing related to IEGM-based events and/or events associated with pressure waveforms, and magnitudes of various IEGM related signals and/or pressure related signals.

In some embodiments the device collects the baseline data over a period of time. That is, parameter acquisition and baseline update operations may be performed repeatedly (e.g., periodically). For example, the device may acquire ischemia related parameters on an hourly basis and update the baseline data once a day. In some cases, the device may simply set the baseline data to the current value of the corresponding parameter. Alternatively, the device may average the baseline data to, for example, filter out any transient conditions that may affect the accuracy of the baseline data.

In practice, the device may obtain the baseline data at virtually any time (e.g., before or after device implant). However, it may be advantageous to acquire the baseline data (and perform the subsequent ischemia monitoring) at certain times and/or under certain conditions. For example, these operations may be performed when the patient is at rest to reduce the influence of the patient's activity on the ischemia detection operation. Accordingly, the device may include one or more sensors (e.g., an activity sensor or a time indicator) that are used to control when these operations are or are not performed.

As represented by block 204, the device will repeatedly (e.g., periodically) monitor the ischemia related parameters. For example, monitoring of these parameters may be invoked every few minutes.

As discussed above in some embodiments the device may monitor IEGM related parameters (block 206). In embodiments where the device is an implantable cardiac device such as an ICD, the device may acquire the IEGM data in conjunction with its normal pacing and other operations. In this case, the IEGM data may be readily available (e.g., stored in a data memory in the ICD).

As represented by block 208, the device also regularly monitors pressure related parameters. For example, the device may store raw data indicative of cardiac chamber pressure measurements acquired over a given time period (e.g., a cardiac cycle). Such data may thus represent, for example, a waveform that tracks the amplitude of the chamber pressure during that time period.

As represented by block 210, the device may process the raw data to generate one or more ischemia-related parameters. For example, the device may identify certain well-known events from the waveforms (e.g., R-waves, c-waves v-waves, etc). The device may then calculate one or more time periods associated with these events or some other aspect of the acquired data.

In some embodiments the device may calculate time periods between events associated with different chambers of the heart. For example, the device may determine whether there is a delay between contraction of the right ventricle and contraction of the left ventricle.

In some embodiments the device may determine certain relative relationships between acquired data. For example, the device may monitor the relative difference between two pressure waveforms over time. In this case, changes in the relative difference between the waveforms may then be used to indicate ischemia.

As represented by block 212, the device determines whether any of the currently acquired parameters (from blocks 206 - 210) are not at expected values. That is, the device determines whether there has been a change in the parameters from, for example, a baseline value or a prior value of the parameter.

In a typical implementation one or more thresholds may be defined for a given parameter. Here, a threshold may be defined such that if an acquired parameter falls above or below the threshold or outside of a range defined by the threshold, an ischemia may be indicated. For example, if a currently detected time period exceeds the previously detected time period by more than a certain amount or a certain percentage (e.g., a percentage of a cardiac cycle) ischemia may be indicated. In addition, a threshold may be set to prevent or reduce the number of false positives.

If the monitored parameters have not changed by an amount sufficient to trigger an ischemia indication, the operations described in FIG. 2 will continue collecting baseline data and monitoring ischemia related parameters. If, on the other hand, the parameters have changed by a sufficient amount, the device will generate an indication of ischemia as discussed above (block 214). Subsequently, the device may continue to perform the operations described in FIG. 2.

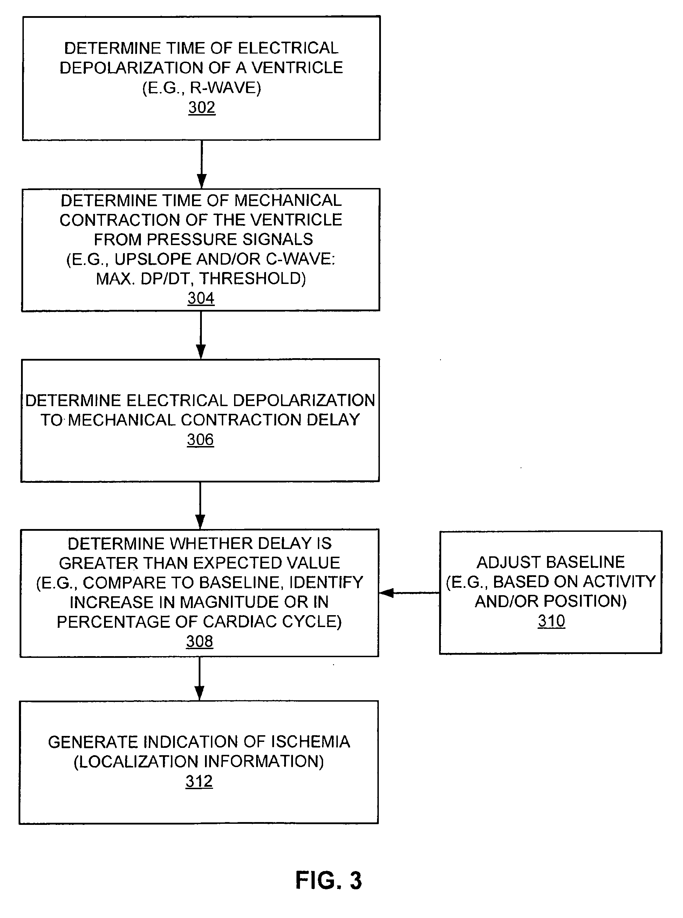

Referring now to FIGS. 3-12, additional details of specific pressure-related ischemia detection operations will be treated. In general, the operations associated with these figures may be performed repeatedly and in conjunction with an overall ischemia detection scheme as discussed above in conjunction with FIGS. 1 and 2.

FIG. 3 relates to operations that may be performed to generate an indication of ischemia based on an increase in an electrical depolarization to mechanical contraction delay in a ventricle. In general, the described operations may be applicable to either the right ventricle or the left ventricle.

As represented by block 302, the device determines the point in time at which electrical depolarization of the ventricle commences. Conventionally, this information may be derived from the R-wave of the IEGM. It should be appreciated, however, that this information or other suitable information may be derived in some other manner.

Various leads may be used to acquire the IEGM. For example, an endocardial lead, an epicardial lead or a pericardial lead may be adapted to sense cardiac electrical activity from a given chamber. In the example of FIG. 13 discussed below, an endocardial lead 1308 is used to acquire IEGM signals from the right ventricle while an epicardially-placed to lead 1306 is used to acquire IEGM signals from the left ventricle.

As represented by block 304, the device then determines the point in time at which mechanical contraction of the ventricle commences. In this case, the device is coupled to a pressure sensor that is adapted to measure pressure, either directly or indirectly, associated with the corresponding chamber. For example, in the example of FIG. 13 the right ventricle lead 1308 includes a pressure sensor 1325 adapted to directly measure pressure in the right ventricle.

In contrast, in the example of FIG. 13 left ventricle pressure information is obtained indirectly via left atrial pressure measurements. For example, a lead 1304 may be adapted to be implanted across the inter-atrial septum such that a pressure sensor 1329 is placed in or near the left atrium. Alternatively, a pressure sensor 1327 on the lead 1306 may be adapted to sense left atrial pressure.

It should be appreciated that the pressure sensors depicted in FIG. 13 are but a few examples of pressure sensing techniques that may be used in accordance with the teachings herein and that pressure measurements may be made in a variety of other ways. For example, pressure sensors may be implanted via some type of approach other than the transvenous approach (e.g., via a pericardial approach).

Moreover, in some applications left ventricular pressure may be measured directly through the use of, for example, a pressure sensor implanted in or near the left ventricle. For example, a pressure sensor may be implanted transvenously into the right ventricle then through the inter-ventricular septum into the left ventricle.

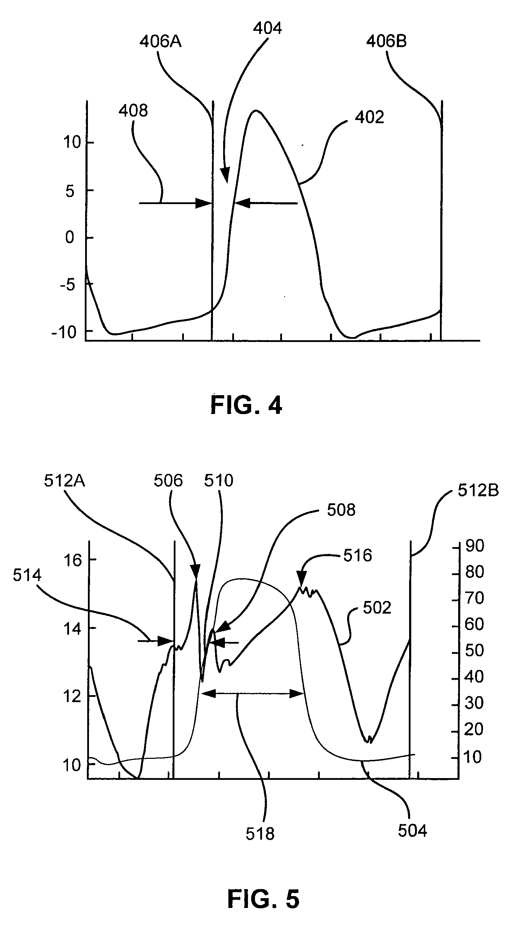

Referring now to FIG. 4, in general, the onset of mechanical contraction relates to an upslope in the ventricular pressure signal. FIG. 4 illustrates an example of a ventricular pressure waveform 402 (e.g., for the right ventricle) where the upslope is indicated by line 404. The waveform 402 is depicted in time relative to the timing of the R-waves in the ventricle as represented by the lines 406A and 406B. Accordingly, an electrical depolarization to mechanical contraction delay may be defined as the period of time between the R-wave 406A and a point on the upslope 404 of the ventricular pressure waveform 402 (block 306 in FIG. 3). One example of such an interval is represented by the arrows 408.

The precise point in time associated with the onset of the mechanical contraction may be defined in various ways. Of importance here is that the same or similar parameter be monitored over time to track changes in this interval that result from ischemia. Preferably, such a parameter would change in a relatively significant manner during ischemia. One example of such a parameter is the maximum change in pressure over time (max. dp/dt) of the ventricular pressure signal. During ischemia the ventricle may not be able to pump as vigorously as normal. Consequently, the maximum change in pressure over time may occur at a point in time that is substantially later than the normal max. dp/dt time.

It should be appreciated that other parameters may be used to define the electrical depolarization to mechanical contraction delay. For example, some embodiments may employ a threshold value (e.g., corresponding to one of the example pressure parameters shown on the left side of the graph) to define a point in time associated with the onset of mechanical contraction. Here, the delay may be defined as the period of time between the R-wave 406A and the point in time at which the upslope 404 crosses the threshold. In other embodiments parameters such as the time of the peak of the waveform 402 may be used to define a point in time associated with mechanical contraction.

As discussed above, the onset of mechanical contraction in the left ventricle may be determined through analysis of left atrial pressure signals. FIG. 5 illustrates an example of a left atrial pressure waveform 502 superimposed on a corresponding left ventricle pressure waveform 504. The left atrial pressure waveform 502 includes several components relating to the cardiac cycle. For example, an a-wave 506 is associated with contraction of the atrium. A c-wave 508 is associated with contraction of the left ventricle. FIG. 5 illustrates that an upslope 510 of the c-wave 508 corresponds, in part, with an upslope of the corresponding left ventricle pressure wave 504. Accordingly, the c-wave may be used to determine the onset of mechanical contraction of the left ventricle.

In FIG. 5 the waveforms 502 and 504 are also shown relative to the timing of the R-waves 512A and 512B for the left ventricle. Accordingly, the electrical depolarization to mechanical contraction delay for the left ventricle may be represented by the arrows 514 between the R-wave 512A and the upslope 510 of the c-wave 508. A particular point in time of the upslope 510 may be selected, for example, as discussed above. That is, the point in time may be defined as a point of maximum dp/dt, may be based on a threshold crossing (e.g., relative to one of the example atrial pressure parameters on the left side of the graph), or may be based on some other suitable factor.

Various techniques may be employed to isolate an a-wave 506, a c-wave 508, or other types of waves (e.g., a v-wave discussed below) from the overall left atrial pressure waveform 502. In some embodiments such features of the waveform 502 may be identified based on the relative timing of an electrocardiogram ("EGM") such as an IEGM and the waveform 502. Each cycle of an EGM waveform, which corresponds to a heart beat, includes a P-wave that is a normally small positive wave caused by the beginning of a heart beat. The P-wave represents atrial depolarization (also known as atrial activation), which initiates contraction of the atrial musculature. Following the P-wave there is a portion of the EGM waveform that is substantially constant in amplitude. The R-wave (representing ventricular depolarization, also known as ventricular activation) of the EGM typically is a rapid positive deflection that occurs after the substantially constant portion. Each cycle of the left atrial pressure waveform includes an a-wave, which is produced by an atrial contraction. Following the a-wave is a c-wave, which is produced by the left ventricle contracting against the closed mitral valve ("MV"). Following the c-wave is a v-wave, which is produced by the left ventricle end systole between the aortic valve closure and the mitral valve opening.

The above features of the left atrial pressure waveform may be identified relative to an event detected in the EGM that corresponds to the pressure signal. Such an event may correspond to, for example, ventricular activation associated with an R-wave (intrinsic activation) or a V-pulse (paced activation). Thus, in the example of FIG. 5, a feature may be expected a given amount of time (e.g., within a given tolerance range) after the R-wave 512A. It should be appreciated that another EGM event may serve as a reference point for identifying features in a left atrial pressure waveform. For example, the P-wave may be sensed and this timing used in conjunction with a known (or an estimate of) the P-R interval or A-V delay to identify a feature that occurs a specified amount of time after a P-wave (e.g., within a given tolerance range).

In some embodiments features of the pressure waveform may be identified through the use of one or more time windows within which a given feature is expected to occur. Several examples of such windows are described below. It should be appreciated, however, that windows may be defined in other ways.

In some embodiments a window may be defined to include at least one of an a-wave and a c-wave, but not a v-wave, of the cardiac cycle represented in the pressure signal. The beginning of such a window may be defined to coincide, for example, with the ventricular activation, as detected based on a detected R-wave or V-pulse. In some embodiments each window length may be a percentage of the previous or upcoming cardiac cycle length (e.g. 30%), or a percentage of the mean of a previous plurality of cardiac cycle lengths (e.g. 30% of the mean of a given number of previous R-wave-to-R-wave intervals), or the length may be a fixed value (e.g. 120 milliseconds). Mechanistically, such a window may correspond to the time after ventricular depolarization (i.e., activation) to the time of ventricular contraction and mitral valve closure.

In some embodiments a window associated with a later part of the cardiac cycle may be defined to include a v-wave, but not an a-wave or a c-wave. Such a window may be defined as starting at the end of the first window. Alternatively, this window may be defined as starting a specified delay after a ventricular or atrial activation. For example, this window may be defined to start 120 milliseconds after a ventricular activation. It is also possible that there may be a small time gap between the first and second windows, or a small overlap between first and second windows. Here, each window length may be a percentage of the previous or upcoming cardiac cycle length (e.g. 70%), or a percentage of the mean of a previous plurality of cardiac cycle lengths (e.g. 70% of the mean of a given number of previous R-wave-to-R-wave intervals), or the length may be a fixed value (e.g. 280 milliseconds). Mechanistically, such a window may correspond to the time after ventricular contraction and mitral valve closure to the time of the next ventricular activation.

Referring again to FIG. 3, as represented by block 308, the device determines whether the delay value determined at block 306 exceeds a value that would be expected in the absence of ischemia. This operation may involve, for example, comparing the delay to a baseline delay, a prior value or some other parameter. The baseline data may be acquired, for example, in a manner as discussed above in conjunction with FIG. 2. For some parameters and under some conditions, the device may simply determine whether the delay has increased in magnitude by a given amount (e.g., in accordance with the threshold) sufficient to indicate an ischemic condition.

Alternatively, the device may determine whether there has been a change in a percentage of time of the cardiac cycle (the time from R-wave 406A to R-wave 406B) associated with the delay. That is, if the delay now occupies a larger percentage of the cardiac cycle, an ischemic condition may be indicated. This type of test may prove advantageous in applications where measurements may be made under a variety of conditions. For example, time intervals associated with the cardiac cycle will generally decrease when the time interval of the cardiac cycle decreases (e.g., as the patient's heart rate increases with exercise). However, for some parameters a relative percentage relationship may hold true regardless of the heart rate. Accordingly, in such a case a deviation in this relative percentage may indicate ischemia.

In some embodiments the device may adjust the baseline, threshold or other parameter based on an activity level, a position, a heart rate, or some other condition associated with the patient (block 310). For example, the device may include a lookup table that defines different baselines, conversion factors for the monitored data, etc., for various activity levels. Alternatively, an algorithm may be applied to the baseline parameter or the monitored data. In any of these cases, the device may include or be coupled to one or more sensors for measuring patient activity, etc., for selecting the appropriate baseline, conversion data, etc.

As represented by block 312, depending upon the results of the comparison at block 308, the device may generate an indication of ischemia. In conjunction with this indication, the device may provide localization information indicating which ventricle is ischemic since the device has the capability of separately monitoring the delay in each ventricle. Accordingly, the specificity of the ischemia indication may be improved as compared to an ischemia indication generated by some other means (e.g., IEGM based).

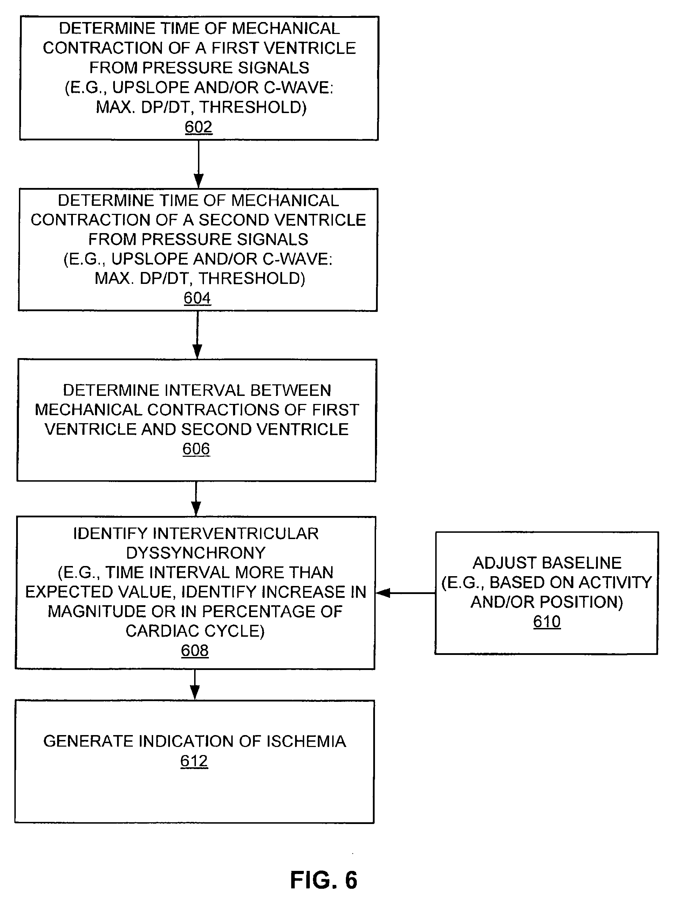

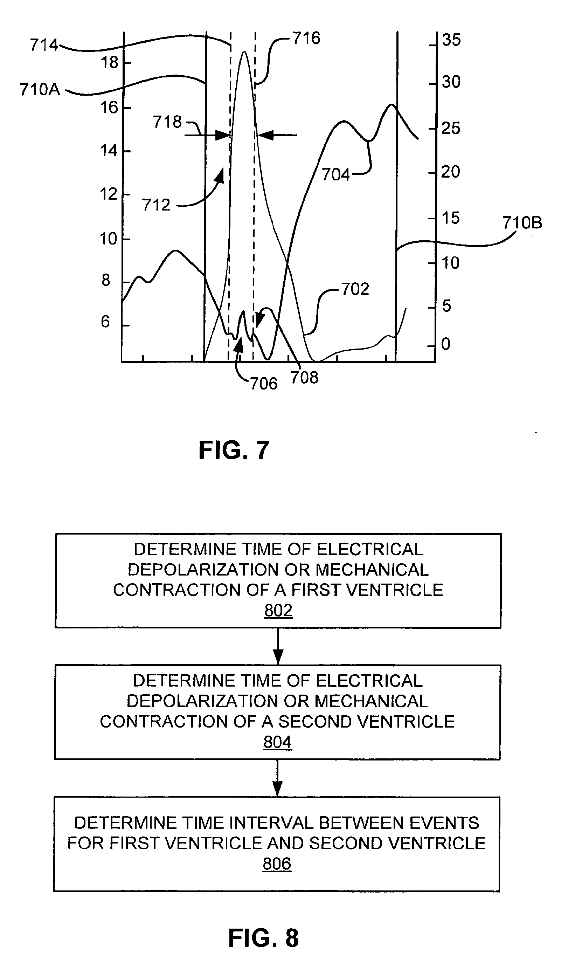

FIGS. 6 - 8 relate to exemplary operations that may be performed to generate an indication of ischemia based on an increase in a time interval between contraction of the right and left ventricles. Specifically, the operations of FIG. 6 relate to detecting this time interval via pressure signals. In contrast, the operations of FIG. 8 relate to detecting this time interval via a combination of electrical and pressure signals.

As represented by block 602 in FIG. 6, the device uses pressure signals to determine the time of mechanical contraction of the first ventricle. This may be accomplished, for example, as discussed above in conjunction with FIG. 3. That is, the device may select a point in time (e.g., using max. dp/dt, a threshold, etc.) on the upslope of a ventricle pressure waveform (e.g., for the right ventricle). Alternatively, the device may select a similar point in time on the upslope of a c-wave representative of the upslope of a pressure waveform for a corresponding ventricle (e.g., the left ventricle).

As represented by block 604, the device uses pressure signals to determine the time of mechanical contraction of the second ventricle. Again, this may be accomplished as discussed above.

As represented by block 606, the device determines the time interval, if any, between the mechanical contractions of the two ventricles. In a healthy heart there may be little or no difference in time between these contractions. However, an ischemic condition (e.g., resulting in a left bundle branch block) may cause a dyssynchrony in the interventricular timing.

FIG. 7 illustrates an example of such an interventricular dyssynchrony. Here, a right ventricle pressure waveform 702 is shown superimposed with a left atrial pressure waveform 704. Left atrial pressure features such as an a-wave and a c-wave are indicated by the arrows 706 and 708, respectively. In addition, the waveforms 702 and 704 are depicted relative to R-waves 710A and 710B for the right ventricle.

As discussed above, the onset of mechanical contraction for the right ventricle corresponds with an upslope 712 of the waveform 702. An example of a point in time corresponding to the upslope 712 is represented by dashed line 714.

Also as discussed above, the onset of mechanical contraction for the left ventricle corresponds to the upslope of the c-wave 708. An example of a point in time corresponding to this upslope is represented by dashed line 716.

Accordingly, in the example of FIG. 7, the time interval between the onsets of mechanical contraction for the two ventricles is represented by arrows 718. Here, it may be seen that the time interval 718 is relatively long with respect to the duration of the cardiac cycle (e.g., the interval is indicative of a left bundle branch block).

Accordingly, as represented by block 608, the device may identify an interventricular dyssynchrony based on the value of this time interval. Here, the device may determine whether the time interval 718 exceeds a value (e.g., close to zero) that would be expected in the absence of ischemia. This operation may involve, for example, comparing the time interval 718 to a baseline time interval, a prior value or some other parameter. The baseline data may be acquired, for example, in a manner as discussed above in conjunction with FIG. 2. In some embodiments the device may determine whether the time interval 718 has increased in magnitude by a given amount (e.g., in accordance with a threshold) sufficient to indicate an ischemic condition. In some embodiments the device may determine whether there has been a change in the percentage of time of the cardiac cycle (the time from R-wave 710A to R-wave 710B) associated with the time interval 718 in a similar manner as discussed above in conjunction with FIG. 3. Alternatively, in some embodiments the device may adjust the baseline, threshold or other parameters in accordance with an activity level, a position, or some other condition associated with the patient in a similar manner as discussed above in conjunction with FIG. 3 (block 610). As represented by block 612, if an interventricular dyssynchrony is indicated at block 608, the device may generate an indication of ischemia.

Figure 8 illustrates an alternative technique for identifying an interventricular dyssynchrony. Here, the device uses a combination of electrical and mechanical events to determine, for example, whether the contraction times of the ventricles are no longer aligned or substantially aligned. For example, referring to block 802 initially the device may determine a time of an electrical depolarization time for the first ventricle (e.g., right ventricle). Then, at block 804 the device may determine a time of a mechanical contraction for the second ventricle (e.g., left ventricle). The device may obtain this timing information as discussed above.

As represented by block 806, the device then determines the time interval between these events. Here, it should be appreciated that in the event of a left bundle branch block or some other similar condition, the mechanical contraction of the left ventricle will be delayed. In this case, the time interval obtained a block 806 will be longer than normal.

The remainder of the process may then follow the operations of blocks 608 - 612 of FIG. 6. In this case, however, the expected time interval (e.g., the baseline) may be longer due to the inherent electromechanical delay.

Blocks 802 and 804 of FIG. 8 also illustrate that in alternate embodiments the device may monitor a mechanical event in the first ventricle (e.g., the right ventricle) while monitoring an electrical event in the second ventricle (e.g., the left ventricle). In such a case, the expected value (e.g., baseline) may be a negative value.

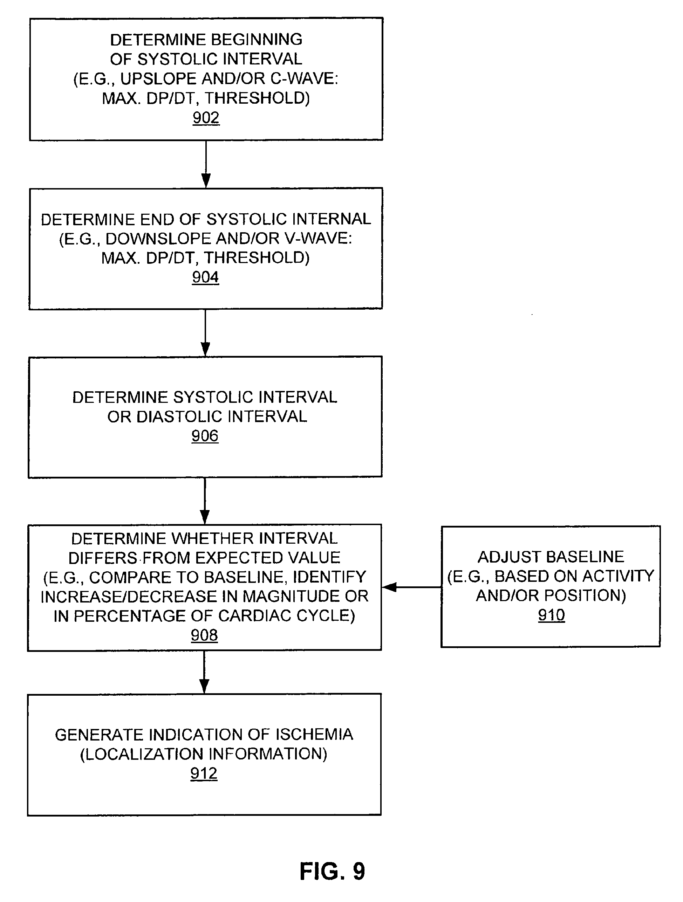

Referring now to FIG. 9, in some embodiments ischemia may be detected based on a shortening of the systolic interval associated with the ventricle. Here, it should be appreciated that a shortening of the systolic interval also corresponds to a lengthening of the diastolic interval. Accordingly, this indicator may be based on either of these parameters.

As represented by block 902, the device uses pressure information to determine the beginning of the systolic interval. This may be accomplished, for example, as discussed above in conjunction with FIG. 3. That is, the device may select a point in time (e.g., using max. dp/dt, a threshold, etc.) on the upslope of a ventricle pressure waveform (e.g., for the right ventricle). Alternatively, the device may select a similar point in time on the upslope of a c-wave representative of the upslope of a pressure waveform for a corresponding ventricle (e.g., the left ventricle).

As represented by block 904, the device uses pressure information to determine the end of the systolic interval. Here, the device may select a point in time (e.g., using max. dp/dt, a threshold, etc.) on the downslope of the ventricle pressure waveform.

Alternatively, for the case where ventricular pressure is obtained indirectly via atrial pressure, the downslope of the ventricle pressure waveform corresponds to the v-wave of the atrial pressure waveform. This relationship is illustrated in FIG. 5, which shows that the downslope of the waveform 504 (e.g., the end of the systolic interval) corresponds to the peak of the v-wave 516.

As represented by block 906, the device may determine the length of the systolic interval based on the difference between the time periods derived at blocks 902 and 904. FIG. 5 illustrates an example of the systolic interval 518. In addition, the device may determine the length of the diastolic interval by subtracting the systolic interval from the cardiac cycle time interval (R-wave 512A to R-wave 512B).

As represented by block 908, the device may determine whether the time interval (e.g., systolic or diastolic interval) associated with the systolic interval 518 is different than a value that would be expected in the absence of ischemia. This operation may involve, for example, comparing the time interval to a baseline time interval, a prior value or some other parameter. The baseline data may be acquired, for example, in a manner as discussed above in conjunction with FIG. 2. In some embodiments the device may determine whether the systolic (diastolic) interval has decreased (increased) in magnitude by a given amount (e.g., in accordance with a threshold) sufficient to indicate an ischemic condition.

In some embodiments the device may determine whether there has been a change in the percentage of time of the cardiac cycle associated with the time interval in a similar manner as discussed above in conjunction with FIG. 3. For example, in general, the systolic interval occupies approximately one third of the total cardiac cycle time. This percentage relationship may be maintained even if the cardiac cycle time decreases due to, for example, an increased level of activity by the patient. Accordingly, a decrease in the systolic interval percentage (or an increase in the diastolic interval percentage) may indicate ischemia.

In some embodiments the device may adjust the baseline, threshold or other parameters based on an activity level, a position, or some other condition associated with the patient (block 910). This may be accomplished, for example, in a similar manner as discussed above in conjunction with FIG. 3.

As represented by block 912, depending upon the results of the comparison at block 908, the device may generate an indication of ischemia. In conjunction with this indication, the device may provide localization information indicating which ventricle is ischemic since the device has the capability of separately monitoring the systolic and diastolic intervals in each ventricle.

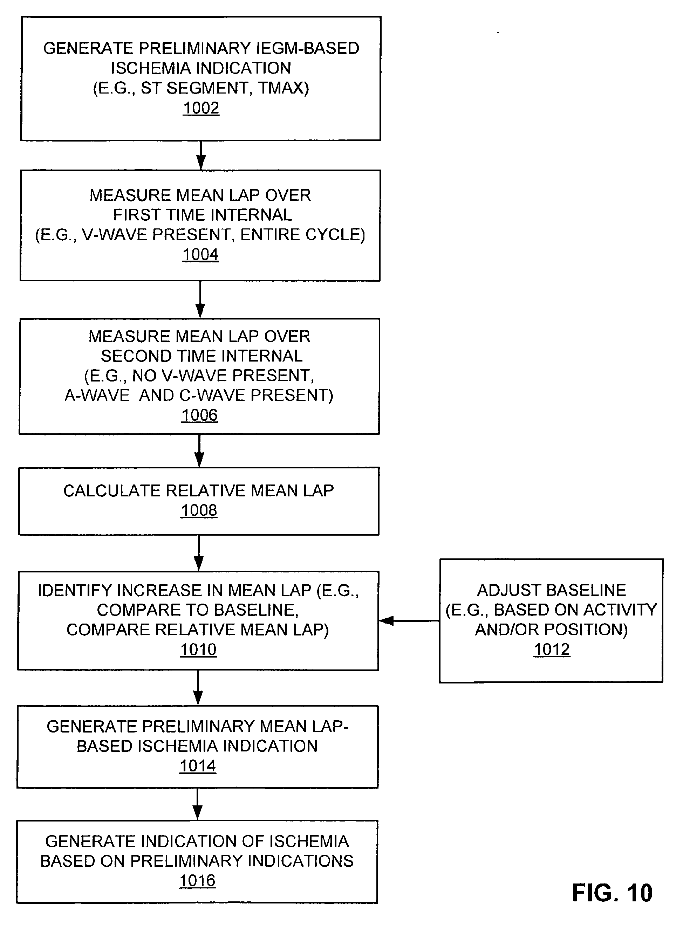

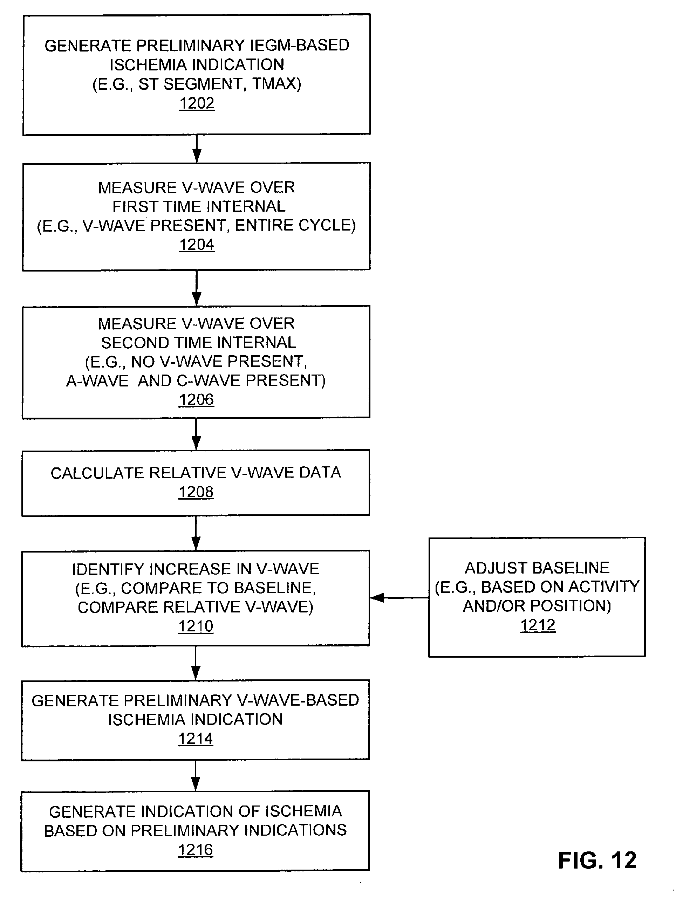

Referring now to FIGS. 10 - 12, in some embodiments a device generates an indication of ischemia based on an IEGM-based ischemia indication and on left atrial pressure measurements. During ischemia there may be a rise in mean left atrial pressure as well as a high occurrence of ischemia-induced mitral valve regurgitation. The regurgitation is generally detectable as an increase in the size (e.g., magnitude) of the v-wave. The operations of FIG. 10 relate to generating an ischemia indication based, in part, on mean left atrial pressure ("LAP") data. The operations of FIG. 12 relate to generating an ischemia indication based, in part, on an enlargement of a v-wave.

As represented by block 1002 in FIG. 10, the device (or some other device) may generate a preliminary IEGM-based ischemia indication. As discussed above, this may involve, for example, a valuation of the level of the ST segment or the timing of the IEGM T-wave.

At blocks 1004 and 1006 the device measures mean left atrial pressure over one or more time intervals. For example, in some embodiments the device detects changes in mean left atrial pressure by comparing the present pressure value with a single baseline or prior value. Alternatively, in some embodiments the device detects changes in mean left atrial pressure by monitoring relative pressure associated with different time intervals (e.g., two or more time intervals). Exemplary operations for the single and multiple time period embodiments will be discussed in turn in conjunction with, respectively, blocks 1004, 1006 and 1010 - 1016 and blocks 1004 - 1016.

In an embodiment where the device measures the pressure over a single time interval, the device may perform one of the operations described in blocks 1004 and 1006. Here, mean atrial pressure may be measured in a variety of ways. For example, mean left atrial pressure may be calculated as a running average over a given time period. Mean left atrial pressure also may be calculated as an average of the a-wave and c-wave portion of the left atrial pressure measurement from consecutive cardiac cycles. Accordingly, as represented by blocks 1004 and 1006 the device may calculate mean atrial pressure over the entire cardiac cycle, over a time period where the a-wave and the c-wave are present or over some other time interval. In practice, mean pressure measurements as discussed herein may be made over several cardiac cycles. In this case, the results of these measurements may be averaged to provide a final mean atrial pressure. Alternatively, the median of these measurements may be determined to provide a final averaged atrial pressure.

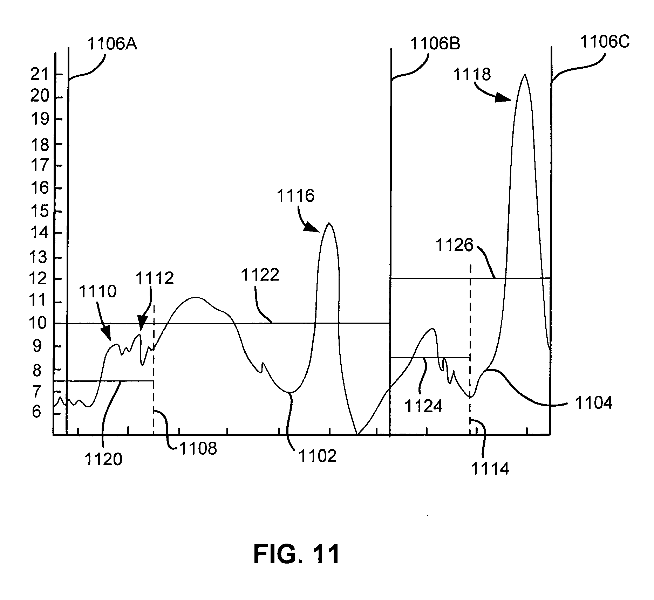

Figure 11 illustrates two left atrial pressure waveforms 1102 and 1104 over two different cardiac cycles. A first cardiac cycle including waveform 1102 begins at R-wave 1106A and ends at R-wave 1106B. For convenience, this cardiac cycle will be referred to as cardiac cycle 1102. A second cardiac cycle including waveform 1104 begins at R-wave 1106B and ends at R-wave 1106C. For convenience, this cardiac cycle will be referred to as cardiac cycle 1104.

FIG. 11 also illustrates several examples of time intervals during which mean atrial pressure measurements may be taken. A first time interval begins at R-wave 1106A and ends at a time represented by dashed line 1108. This time interval includes an a-wave 1110 and a c-wave 1112 of the waveform 1102. For convenience, this time interval will be referred to as time interval 1108.

A second time interval begins at R-wave 1106B and ends at a time represented by a dashed line 1114. This time interval includes an a-wave and a c-wave of the waveform 1104. For convenience, this time interval will be referred to as time interval 1114.

The time intervals 1108 and 1114 do not include v-waves 1116 and 1118 of the corresponding waveforms 1102 and 1104. However, the v-waves 1116 and 1118 are included in the time intervals defined by the respective cardiac cycles 1102 and 1104.

FIG. 11 also illustrates several examples of mean pressure values (e.g., relative to the example pressure parameters on the left side of the graph) associated with each time interval. Specifically, a horizontal line 1120 represents a mean pressure for time period 1108. A horizontal line 1122 represents a mean pressure for cardiac cycle 1102. A horizontal line 1124 represents a mean pressure for time period 1114. A horizontal line 1126 represents a mean pressure for cardiac cycle 1104.

Referring now to block 1010 in FIG. 10, the device may determine whether the monitored mean pressure is different than a value that would be expected in the absence of ischemia. This operation may involve, for example, comparing the mean pressure to a baseline mean pressure, a prior value or some other parameter. The baseline data may be acquired, for example, in a manner as discussed above in conjunction with FIG. 2.

In some embodiments the device may adjust the baseline, threshold or other parameters based on an activity level, a position, or some other condition associated with the patient (block 1012). This may be accomplished, for example, in a similar manner as discussed above in conjunction with FIG. 3.

In some embodiments the device may determine whether the mean pressure has increased in magnitude by a given amount (e.g., in accordance with a threshold) sufficient to indicate an ischemic condition. For example, in FIG. 11 the waveform 1102 may represent the left atrial pressure waveform collected at a prior point in time while the waveform 1104 represents the current left atrial pressure waveform. Thus, a current mean pressure (mean pressure 1126) measured over the entire current cardiac cycle 1104 may be compared with the prior mean pressure (mean pressure 1122) measured over the previous cardiac cycle 1102. Alternatively, in embodiments where the mean pressure is measured over the a-wave and c-wave time intervals 1108 and 1114, the device may compare a current mean pressure 1124 with a prior mean pressure 1120. In either case it may be seen in FIG. 11 that in this example the current mean pressure is higher than the prior mean pressure.

As represented by block 1014, based on the results of the comparison at block 1010, the device may generate a preliminary indication of ischemia. At block 1016 the device may then generate an ultimate (e.g., final) indication of ischemia based on the preliminary ischemia indications from blocks 1002 and 1014.

Referring again to the embodiment where the device detects changes in mean left atrial pressure by monitoring relative pressure associated with different time intervals, the device may perform operations associated with both blocks 1004 and 1006 (and, optionally, other similar blocks). For example, the device may measure a first mean pressure over a first time period (block 1004) and measure a second mean pressure over a second time period (block 1006). In the example of FIG. 11, the first time period may comprise the entire cardiac cycle (e.g., cardiac cycle 1104) while the second time period may comprise the a-wave and c-wave portion of the entire cardiac cycle (e.g., time period 1114).

As represented by block 1008, the device may then determine a relative mean pressure associated with the first and second means pressures. That is, the device may determine the difference between the first and second mean pressures. In the example of the prior paragraph, the device may thus calculate the difference between mean pressure 1126 and mean pressure 1124. One potential advantage of the use of relative pressures is that other factors that influence the pressure readings (e.g., respiration) may be cancelled out or reduced.

As represented by block 1010, the device monitors relative mean pressure over time to determine whether a change in this relative mean pressure is indicative of ischemia. Here, the device may determine whether the monitored relative mean pressure is different than a value that would be expected in the absence of ischemia. This operation may involve, for example, comparing the current relative mean pressure to a baseline relative mean pressure, a prior value or some other parameter. The baseline data may be acquired, for example, in a manner as discussed above in conjunction with FIG. 2. Again, the device may adjust the baseline, threshold or other parameters based on an activity level, a position, or some other condition associated with the patient (block 1012).

In some embodiments the device may determine whether the relative mean pressure has increased in magnitude by a given amount (e.g., in accordance with a threshold) sufficient to indicate an ischemic condition. For example, in FIG. 11 the waveform 1102 may represent the left atrial pressure waveform collected at a prior point in time while the waveform 1104 represents the current left atrial pressure waveform. Thus, a current relative mean pressure (mean pressure 1126 minus mean pressure 1124) for the current cardiac cycle 1104 may be compared with the prior relative mean pressure (mean pressure 1122 minus mean pressure 1120) for the previous cycle 1102. It may be seen in FIG. 11 that the current relative mean pressure in this example is larger than the prior relative mean pressure.

As represented by block 1014, based on the results of the comparison at block 1010, the device may generate a preliminary indication of ischemia. The device may then generate an ultimate indication of ischemia based on the preliminary ischemia indications from blocks 1002 and 1014 (block 1016).

FIG. 12 relates to an embodiment where a change in the size of the v-wave provides a supplemental ischemia indicator. The operations of FIG. 12 may be similar to the operations of FIG. 10. For example, the operations of block 1202 may be similar to the operations of block 1002. In addition, in some embodiments the device may compare a current size of a v-wave with a baseline or prior v-wave value. Alternatively, the device may acquire and compare relative pressure measurements. Exemplary operations for these two embodiments will be discussed in turn in conjunction with, respectively, blocks 1204 and 1210 - 1216 and blocks 1204 - 1216.

At block 1204 the device obtains data representative of the size of the v-wave. For example, the device may measure pressure over a time interval that includes the v-wave. With respect to v-wave 1118 in FIG. 11, such a time interval may comprise the entire cardiac cycle 1104 or a time interval that excludes the a-wave and the c-wave (e.g., the time period from line 1114 to R-wave 1106C). It should be appreciated based on the teachings herein that other data relating to the v-wave or some other pressure signal may be used to identify ischemia related to, for example, mitral valve regurgitation.

At block 1210 the device may determine whether the current v-wave data is different than a value that would be expected in the absence of ischemia. This operation may involve, for example, comparing the current v-wave data to baseline v-wave data, a prior value or some other parameter. The baseline data may be acquired, for example, in a manner as discussed above in conjunction with FIG. 2. Again, the device may adjust the baseline, threshold or other parameters based on an activity level, a position, or some other condition associated with the patient (block 1212).

In some embodiments the device may determine whether the v-wave data has increased in magnitude by a given amount (e.g., in accordance with a threshold) sufficient to indicate an ischemic condition. For example, in FIG. 11 the waveform 1102 may represent the left atrial pressure waveform collected at a prior point in time while the waveform 1104 represents the current left atrial pressure waveform. Thus, a current mean pressure taken over a time period that includes the v-wave (e.g., mean pressure 1126) may be compared with a prior mean pressure taken over a comparable time period (e.g., mean pressure 1122). Here, it may be seen that in this example the current mean pressure is higher than the prior mean pressure, thereby indicating an increase in the size of the v-wave.

As represented by block 1214, based on the results of the comparison at block 1210, the device may generate a preliminary indication of ischemia. At block 1216 the device may thus generate an ultimate indication of ischemia based on the preliminary ischemia indications from blocks 1202 and 1214.

Referring now to the embodiment where the device utilizes relative pressure measurements, these operations may be similar to the relative mean pressure monitoring operations discussed above in conjunction with FIG. 10. For example, the pressure measurements 1122 and 1126 include pressure components related to the v-waves 1116 and 1118, respectively. In contrast, the pressure measurements 1120 and 1124 do not include pressure components related to the v-waves 1116 and 1118. Such a distinction may be used to obtain relative pressure data between a pressure measurement that include a v-wave component and a pressure measurement that does not include a v-wave component. Specifically, in the event the v-wave increases in size (e.g., v-wave 1118), the former pressure measurement (e.g., pressure 1126) will increase while the latter pressure measurement (e.g., pressure 1124) may not increase significantly. This increase in relative pressure may thus provide an indication of ischemia (e.g., ischemia that caused mitral valve regurgitation).

Accordingly, as represented by blocks 1204 and 1206, the device may obtain pressure data over a first time period and a second time period. Here, the first time period may include a v-wave component while the second time period does not.

As represented by block 1208 the device then calculates relative v-wave data for the two sets of pressure data. For example, the device may subtract one set of pressure data (e.g., pressure 1126) from the other (e.g., pressure 1124).

As represented by block 1210 the device determines whether there has been an increase in the relative pressure data. For example, the current relative data (from block 1208) may be compared with baseline data, a threshold, etc., that may be adjusted based on one or more factors (block 1212). As a specific example, the current relative data (e.g., pressure 1126 minus pressure 1124) may be compared with prior relative data (e.g., pressure 1122 minus pressure 1120). As FIG. 11 illustrates, in this example the relative pressure data has increased, thereby indicating a potential ischemic condition. The device may then generate a preliminary pressure-based ischemia indication (block 1214) and an ultimate ischemia indication based on the preliminary indications (block 1216).

The operations discussed above may be implemented in a variety of ways and using various components. For example, some or all of these operations may be performed in an implantable device. FIG. 13 illustrates an example of an implantable cardiac device (e.g., a stimulation device such as an implantable cardioverter defibrillator, a pacemaker, etc.) that is capable of being used in connection with the various embodiments that are described herein. It is to be appreciated and understood that other cardiac devices, including those that are not necessarily implantable, can be used and that the description below is given, in its specific context, to assist the reader in understanding, with more clarity, the embodiments described herein.

FIG. 13 shows an exemplary implantable cardiac device 1300 in electrical communication with a patient's heart H by way of three leads 1304, 1306 and 1308, suitable for delivering multi-chamber stimulation and shock therapy. To sense atrial cardiac signals and to provide right atrial chamber stimulation therapy, the device 1300 is coupled to an implantable right atrial lead 1304 having, for example, an atrial electrode 1320 (referred to herein as the atrial tip electrode), which in this example is implanted in the patient's right atrial septum. FIG. 13 also shows the right atrial lead 1304 as having an optional atrial ring electrode 1321.

To sense left atrial and ventricular cardiac signals and to provide left chamber pacing therapy, device 1300 is coupled to a coronary sinus lead 1306 designed for placement in the coronary sinus region via the coronary sinus for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, an exemplary coronary sinus lead 1306 is designed to receive atrial and ventricular cardiac signals and to deliver left ventricular pacing therapy using, for example, a left ventricular tip electrode 1322 and, optionally, a left ventricular ring electrode 1323; provide left atrial pacing therapy using, for example, a left atrial ring electrode 1324; and provide shocking therapy using, for example, a left atrial coil electrode 1326 (or other electrode capable of delivering a shock). For a more detailed description of a coronary sinus lead, the reader is directed to U.S. Patent No. 5,466,254, "Coronary Sinus Lead with Atrial Sensing Capability" (Helland), which is incorporated herein by reference.

Device 1300 is also shown in electrical communication with the patient's heart H by way of an implantable right ventricular lead 1308 having, in this implementation, a right ventricular tip electrode 1328, a right ventricular ring electrode 1330, a right ventricular (RV) coil electrode 1332 (or other electrode capable of delivering a shock), and superior vena cava (SVC) coil electrode 1334 (or other electrode capable of delivering a shock). Typically, the right ventricular lead 1308 is transvenously inserted into the heart H to place the right ventricular tip electrode 1328 in the right ventricular apex so that the RV coil electrode 1332 will be positioned in the right ventricle and the SVC coil electrode 1334 will be positioned in the superior vena cava. Accordingly, the right ventricular lead 1308 is capable of sensing or receiving cardiac signals, and delivering stimulation in the form of pacing and shock therapy to the right ventricle.

The leads 1304, 1306, and 1308 also may include one or more pressure sensors for measuring pressure in a corresponding chamber of the heart H. For example, a pressure sensor 1325 on the lead 1308 may measure pressure in the right ventricle. In addition, a pressure sensor 1327 on the lead 1306 may be adapted to measure pressure in the left atrium.

In a typical embodiment, the right atrial lead 1304 or some other lead may be implanted in the septal wall (e.g., in the area of the fossa ovalis) separating the right atrium and the left atrium to measure pressure in the left atrium. For example, the lead 1304 may include a pressure sensor 1329 located on a distal portion of the lead. Alternatively, the pressure sensor 1329 may be located at some other location along the lead and coupled to receive pressure waves from the left atrium.

It should be appreciated that other mechanisms may be employed to measure pressure in a chamber or some other vessel. For example, a sensor on a lead routed to the right ventricle may be implanted across the septal wall separating the left and right ventricles. Such a sensor may thus be used to directly measure left ventricular pressure. Here, however, precautions may need to be taken to reduce the risks that may be associated with implanting a foreign object in the left ventricle.

Device 1300 is also shown in electrical communication with a lead 1310 including one or more components 1344 such as a physiologic sensor. The lead 1310 may be positioned in, near or remote from the heart.

It should be appreciated that the device 1300 may connect to leads other than those specifically shown. In addition, the leads connected to the device 1300 may include components other than those specifically shown. For example, a lead may include other types of electrodes, sensors or devices that serve to otherwise interact with a patient or the surroundings.

FIG. 14 shows an exemplary, simplified block diagram depicting various components of the cardiac device 1300. The device 1300 may be adapted to treat both fast and slow arrhythmias with stimulation therapy, including cardioversion, defibrillation, and pacing stimulation. While a particular multi-chamber device is shown, it is to be appreciated and understood that this is done for illustration purposes. Thus, the techniques and methods described below can be implemented in connection with any suitably configured or configurable device. Accordingly, one of skill in the art could readily duplicate, eliminate, or disable the appropriate circuitry in any desired combination to provide a device capable of treating the appropriate chamber(s) with, for example, cardioversion, defibrillation, and pacing stimulation.

Housing 1400 for device 1300 is often referred to as the "can", "case" or "case electrode", and may be programmably selected to act as the return electrode for all "unipolar" modes. Housing 1400 may further be used as a return electrode alone or in combination with one or more of the coil electrodes 1326, 1332 and 1334 for shocking purposes. Housing 1400 further includes a connector (not shown) having a plurality of terminals 1401, 1402, 1404, 1405, 1406, 1408, 1412, 1414, 1416 and 1418 (shown schematically and, for convenience, the names of the electrodes to which they are connected are shown next to the terminals).

The connector may be configured to include various other terminals depending on the requirements of a given application. For example, the device 1300 may include one or more terminals that are coupled to receive pressure signals from one or more pressure sensors (e.g., provided on the leads 1304, 1306 and 1308 or in some other manner). In the specific example of FIG. 14, the device 1300 includes a terminal 1421 for receiving left side pressure signals (e.g., from sensor 1329). In addition, the device 1300 includes a terminal 1423 for receiving right side pressure signals (e.g., from sensor 1325).

To achieve right atrial sensing and pacing, the connector includes, for example, a right atrial tip terminal (AR TIP) 1402 adapted for connection to the atrial tip electrode 1320. A right atrial ring terminal (AR RING) 1401 may also be included and adapted for connection to the atrial ring electrode 1321. To achieve left chamber sensing, pacing, and shocking, the connector includes, for example, a left ventricular tip terminal (VL TIP) 1404, a left ventricular ring terminal (VL RING) 1405, a left atrial ring terminal (AL RING) 1406, and a left atrial shocking terminal (AL COIL) 1408, which are adapted for connection to the left ventricular tip electrode 1322, the left ventricular ring electrode 1323, the left atrial ring electrode 1324, and the left atrial coil electrode 1326, respectively.

To support right chamber sensing, pacing, and shocking, the connector further includes a right ventricular tip terminal (VR TIP) 1412, a right ventricular ring terminal (VR RING) 1414, a right ventricular shocking terminal (RV COIL) 1416, and a superior vena cava shocking terminal (SVC COIL) 1418, which are adapted for connection to the right ventricular tip electrode 1328, the right ventricular ring electrode 1330, the RV coil electrode 1332, and the SVC coil electrode 1334, respectively.

At the core of the device 1300 is a programmable microcontroller 1420 that controls the various modes of stimulation therapy. As is well known in the art, microcontroller 1420 typically includes a microprocessor, or equivalent control circuitry, designed specifically for controlling the delivery of stimulation therapy, and may further include memory such as RAM, ROM and flash memory, logic and timing circuitry, state machine circuitry, and I/O circuitry. Typically, microcontroller 1420 includes the ability to process or monitor input signals (data or information) as controlled by a program code stored in a designated block of memory. The type of microcontroller is not critical to the described implementations. Rather, any suitable microcontroller 1420 may be used that carries out the functions described herein. The use of microprocessor-based control circuits for performing timing and data analysis functions are well known in the art.

Representative types of control circuitry that may be used in connection with the described embodiments can include the microprocessor-based control system of U.S. Patent No. 4,940,052 (Mann et al.), the state-machine of U.S. Patent Nos. 4,712,555 (Thornander et al.) and 4,944,298 (Sholder), all of which are incorporated by reference herein. For a more detailed description of the various timing intervals that may be used within the device and their inter-relationship, see U.S. Patent 4,788,980 (Mann et al.), also incorporated herein by reference.

FIG. 14 also shows an atrial pulse generator 1422 and a ventricular pulse generator 1424 that generate pacing stimulation pulses for delivery by the right atrial lead 1304, the coronary sinus lead 1306, and/or the right ventricular lead 1308 via an electrode configuration switch 1426. It is understood that in order to provide stimulation therapy in each of the four chambers of the heart, the atrial and ventricular pulse generators 1422 and 1424 may include dedicated, independent pulse generators, multiplexed pulse generators, or shared pulse generators. The pulse generators 1422 and 1424 are controlled by the microcontroller 1420 via appropriate control signals 1428 and 1430, respectively, to trigger or inhibit the stimulation pulses.

Microcontroller 1420 further includes timing control circuitry 1432 to control the timing of the stimulation pulses (e.g., pacing rate, atrio-ventricular (A-V) delay, atrial interconduction (A-A) delay, or ventricular interconduction (V-V) delay, etc.) or other operations, as well as to keep track of the timing of refractory periods, blanking intervals, noise detection windows, evoked response windows, alert intervals, marker channel timing, etc., as known in the art.

Microcontroller 1420 further includes an arrhythmia detector (not shown). The arrhythmia detector may be utilized by the device 1300 for determining desirable times to administer various therapies. The arrhythmia detector may be implemented, for example, in hardware as part of the microcontroller 1420, or as software/firmware instructions programmed into the device and executed on the microcontroller 1420 during certain modes of operation.

Microcontroller 1420 may include a morphology discrimination module 1436, a capture detection module (not shown) and an auto sensing module (not shown). These modules are optionally used to implement various exemplary recognition algorithms and/or methods. The aforementioned components may be implemented, for example, in hardware as part of the microcontroller 1420, or as software/firmware instructions programmed into the device and executed on the microcontroller 1420 during certain modes of operation.

The electrode configuration switch 1426 includes a plurality of switches for connecting the desired terminals (e.g., that are connected to electrodes, coils, sensors, etc.) to the appropriate I/O circuits, thereby providing complete terminal and, hence, electrode programmability. Accordingly, switch 1426, in response to a control signal 1442 from the microcontroller 1420, may be used to determine the polarity of the stimulation pulses (e.g., unipolar, bipolar, combipolar, etc.) by selectively closing the appropriate combination of switches (not shown) as is known in the art.

Atrial sensing circuits (ATR. SENSE) 1444 and ventricular sensing circuits (VTR. SENSE) 1446 may also be selectively coupled to the right atrial lead 1304, coronary sinus lead 1306, and the right ventricular lead 1308, through the switch 1426 for detecting the presence of cardiac activity in each of the four chambers of the heart. Accordingly, the atrial and ventricular sensing circuits 1444 and 1446 may include dedicated sense amplifiers, multiplexed amplifiers, or shared amplifiers. Switch 1426 determines the "sensing polarity" of the cardiac signal by selectively closing the appropriate switches, as is also known in the art. In this way, the clinician may program the sensing polarity independent of the stimulation polarity. The sensing circuits (e.g., circuits 1444 and 1446) are optionally capable of obtaining information indicative of tissue capture.

Each sensing circuit 1444 and 1446 preferably employs one or more low power, precision amplifiers with programmable gain and/or automatic gain control, bandpass filtering, and a threshold detection circuit, as known in the art, to selectively sense the cardiac signal of interest. The automatic gain control enables the device 1300 to deal effectively with the difficult problem of sensing the low amplitude signal characteristics of atrial or ventricular fibrillation.

The outputs of the atrial and ventricular sensing circuits 1444 and 1446 are connected to the microcontroller 1420, which, in turn, is able to trigger or inhibit the atrial and ventricular pulse generators 1422 and 1424, respectively, in a demand fashion in response to the absence or presence of cardiac activity in the appropriate chambers of the heart. Furthermore, as described herein, the microcontroller 1420 is also capable of analyzing information output from the sensing circuits 1444 and 1446 and/or a data acquisition system 1452. This information may be used to determine or detect whether and to what degree tissue capture has occurred and to program a pulse, or pulses, in response to such determinations. The sensing circuits 1444 and 1446, in turn, receive control signals over signal lines 1448 and 1450 from the microcontroller 1420 for purposes of controlling the gain, threshold, polarization charge removal circuitry (not shown), and the timing of any blocking circuitry (not shown) coupled to the inputs of the sensing circuits 1444 and 1446 as is known in the art.

For arrhythmia detection, the device 1300 utilizes the atrial and ventricular sensing circuits 1444 and 1446 to sense cardiac signals to determine whether a rhythm is physiologic or pathologic. It should be appreciated that other components may be used to detect arrhythmia depending on the system objectives. In reference to arrhythmias, as used herein, "sensing" is reserved for the noting of an electrical signal or obtaining data (information), and "detection" is the processing (analysis) of these sensed signals and noting the presence of an arrhythmia.

Timing intervals between sensed events (e.g., P-waves, R-waves, and depolarization signals associated with fibrillation) may be classified by the arrhythmia detector of the microcontroller 1420 by comparing them to a predefined rate zone limit (e.g., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy"). Similar rules may be applied to the atrial channel to determine if there is an atrial tachyarrhythmia or atrial fibrillation with appropriate classification and intervention.

Cardiac signals or other signals may be applied to inputs of an analog-to-digital (A/D) data acquisition system 1452. The data acquisition system 1452 is configured (e.g., via signal line 1456) to acquire intracardiac electrogram ("IEGM") signals or other signals, convert the raw analog data into a digital signal, and store the digital signals for later processing and/or telemetric transmission to an external device 1454. For example, the data acquisition system 1452 may be coupled to the right atrial lead 1304, the coronary sinus lead 1306, the right ventricular lead 1308 and other leads through the switch 1426 to sample cardiac signals across any pair of desired electrodes.

The data acquisition system 1452 also may be coupled to receive signals from other input devices. For example, the data acquisition system 1452 may sample signals from a physiologic sensor 1470 or other components shown in FIG. 14 (connections not shown).

The microcontroller 1420 is further coupled to a memory 1460 by a suitable data/address bus 1462, wherein the programmable operating parameters used by the microcontroller 1420 are stored and modified, as required, in order to customize the operation of the device 1300 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart H within each respective tier of therapy. One feature of the described embodiments is the ability to sense and store a relatively large amount of data (e.g., from the data acquisition system 1452), which may then be used for subsequent analysis to guide the programming of the device.

Advantageously, the operating parameters of the implantable device 1300 may be non-invasively programmed into the memory 1460 through a telemetry circuit 1464 in telemetric communication via communication link 1466 with the external device 1454, such as a programmer, transtelephonic transceiver, a diagnostic system analyzer or some other device. The microcontroller 1420 activates the telemetry circuit 1464 with a control signal (e.g., via bus 1468). The telemetry circuit 1464 advantageously allows intracardiac electrograms and status information relating to the operation of the device 1300 (as contained in the microcontroller 1420 or memory 1460) to be sent to the external device 1454 through an established communication link 1466.

The device 1300 can further include one or more physiologic sensors 1470. In some embodiments the device 1300 may include a "rate-responsive" sensor that may provide, for example, information to aid in adjustment of pacing stimulation rate according to the exercise state of the patient. One or more physiologic sensors 1470 (e.g., a pressure sensor) may further be used to detect changes in cardiac output, changes in the physiological condition of the heart, or diurnal changes in activity (e.g., detecting sleep and wake states). Accordingly, the microcontroller 1420 responds by adjusting the various pacing parameters (such as rate, AV Delay, V-V Delay, etc.) at which the atrial and ventricular pulse generators 1422 and 1424 generate stimulation pulses.

While shown as being included within the device 1300, it is to be understood that a physiologic sensor 1470 may also be external to the device 1300, yet still be implanted within or carried by the patient. Examples of physiologic sensors that may be implemented in conjunction with device 1300 include sensors that sense respiration rate, pH of blood, ventricular gradient, oxygen saturation, blood pressure and so forth. Another sensor that may be used is one that detects activity variance, wherein an activity sensor is monitored diurnally to detect the low variance in the measurement corresponding to the sleep state. For a more detailed description of an activity variance sensor, the reader is directed to U.S. Patent No. 5,476,483 (Bornzin et al.), issued 12/19/1995, which patent is hereby incorporated by reference.

The one or more physiologic sensors 1470 may optionally include sensors to help detect movement (via, e.g., a position sensor) and/or minute ventilation (via an MV sensor) in the patient. Signals generated by the position sensor and MV sensor may be passed to the microcontroller 1420 for analysis in determining whether to adjust the pacing rate, etc. The microcontroller 1420 may thus monitor the signals for indications of the patient's position and activity status, such as whether the patient is climbing up stairs or descending down stairs or whether the patient is sitting up after lying down.

The device 1300 additionally includes a battery 1476 that provides operating power to all of the circuits shown in FIG. 14. For a device 1300 which employs shocking therapy, the battery 1476 is capable of operating at low current drains (e.g., preferably less than 10 µA) for long periods of time, and is capable of providing high-current pulses (for capacitor charging) when the patient requires a shock pulse (e.g., preferably, in excess of 2 A, at voltages above 200 V, for periods of 10 seconds or more). The battery 1476 also desirably has a predictable discharge characteristic so that elective replacement time can be detected. Accordingly, the device 1300 preferably employs lithium or other suitable battery technology.

The device 1300 can further include magnet detection circuitry (not shown), coupled to the microcontroller 1420, to detect when a magnet is placed over the device 1300. A magnet may be used by a clinician to perform various test functions of the device 1300 and/or to signal the microcontroller 1420 that the external device 1454 is in place to receive data from or transmit data to the microcontroller 1420 through the telemetry circuit 1464.

The device 1300 further includes an impedance measuring circuit 1478 that is enabled by the microcontroller 1420 via a control signal 1480. The known uses for an impedance measuring circuit 1478 include, but are not limited to, lead impedance surveillance during the acute and chronic phases for proper performance, lead positioning or dislodgement; detecting operable electrodes and automatically switching to an operable pair if dislodgement occurs; measuring respiration or minute ventilation; measuring thoracic impedance for determining shock thresholds; detecting when the device 1300 has been implanted; measuring stroke volume; and detecting the opening of heart valves, etc. The impedance measuring circuit 1478 is advantageously coupled to the switch 1426 so that any desired electrode may be used.

In the case where the device 1300 is intended to operate as an implantable cardioverter/defibrillator (ICD) device, it detects the occurrence of an arrhythmia, and automatically applies an appropriate therapy to the heart aimed at terminating the detected arrhythmia. To this end, the microcontroller 1420 further controls a shocking circuit 1482 by way of a control signal 1484. The shocking circuit 1482 generates shocking pulses of low (e.g., up to 0.5 J), moderate (e.g., 0.5 J to 10 J), or high energy (e.g., 11 J to 40 J), as controlled by the microcontroller 1420. Such shocking pulses are applied to the patient's heart H through, for example, two shocking electrodes and as shown in this embodiment, selected from the left atrial coil electrode 1326, the RV coil electrode 1332, and/or the SVC coil electrode 1334. As noted above, the housing 1400 may act as an active electrode in combination with the RV coil electrode 1332, and/or as part of a split electrical vector using the SVC coil electrode 1334 or the left atrial coil electrode 1326 (i.e., using the RV electrode as a common electrode).

Cardioversion level shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5 J to 40 J), delivered asynchronously (since R-waves may be too disorganized), and pertaining exclusively to the treatment of fibrillation. Accordingly, the microcontroller 1420 is capable of controlling the synchronous or asynchronous delivery of the shocking pulses.

The device 1300 also includes or operates in conjunction with components that may provide functionality relating to detecting ischemia by obtaining and processing pressure signals as taught herein. Here, signals from a pressure sensor (e.g., received at terminals 1421 and 1423) may be coupled by the switch 1426 or some other mechanism to the data acquisition system 1452. The resulting digitized signals may then be provided to the microcontroller 1420.

The microcontroller 1420 is adapted to implement several functional components relating to processing the pressure signals as taught herein. To this end, the microcontroller may comprise a signal processor or incorporate signal processing or other suitable functionality to implement these functional components. For example, an intraventricular electromechanical delay detector component 1437 may be adapted to provide functionality related to the operations discussed above in conjunction with FIG. 3. An intraventricular dyssynchrony detector component 1438 may be adapted to provide functionality related to the operations discussed above in conjunction with FIGS. 6 and 8. A systolic interval detector component 1439 may be adapted to provide functionality related to the operations discussed above in conjunction with FIG. 9. A mean left atrial pressure and/or v-wave detector component 1410 may be adapted to provide functionality related to the operations discussed above in conjunction with FIGS. 10 and 12. In addition, the device 1300 may include a warning/therapy module 1434 adapted to generate warning signals and/or administer therapy based on analysis of the cardiac condition of the patient.

It should be appreciated that various modifications may be incorporated into the disclosed embodiments based on the teachings herein. For example, the structure and functionality taught herein may be incorporated into types of devices other than those types specifically described. In addition, the various signals described herein and/or other signals may be sensed in other ways and using different sensing components. Such sensors (e.g., electrodes, physiologic sensors, etc.) also may be incorporated into other types of implantable leads or may be implanted or otherwise provided without the use of leads. These sensors may be located at various positions throughout the heart or the body. Various algorithms and/or techniques may be employed to obtain pressure measurements.

Different embodiments of the stimulation device may include a variety of hardware and software processing components. In some embodiments, hardware components such as processors, controllers, state machines and/or logic may be used to implement the described components or circuits. In some embodiments, code including instructions (e.g., software, firmware, middleware, etc.) may be executed on one or more processing devices to implement one or more of the described functions or components. The code and associated components (e.g., data structures and other components by the code or to execute the code) may be stored in an appropriate data memory that is readable by a processing device (e.g., commonly referred to as a computer-readable medium).

Moreover, some of the operations described herein may be performed by a device that is located externally with respect to the body of the patient. For example, an implanted device may simply send raw data or processed data to an external device that then performs the necessary processing.

The components and functions described herein may be connected and/or coupled in many different ways. The manner in which this is done may depend, in part, on whether and how the components are separated from the other components. In some embodiments some of the connections and/or couplings represented by the lead lines in the drawings may be in an integrated circuit, on a circuit board or implemented as discrete wires or in other ways.

The signals discussed herein may take various forms. For example, in some embodiments a signal may comprise electrical signals transmitted over a wire, light pulses transmitted through an optical medium such as an optical fiber or air, or RF waves transmitted through a medium such as air, etc. In addition, a plurality of signals may be collectively referred to as a signal herein. The signals discussed above also may take the form of data. For example, in some embodiments an application program may send a signal to another application program. Such a signal may be stored in a data memory.

Moreover, the recited order of the blocks in the processes disclosed herein is simply an example of a suitable approach. Thus, operations associated with such blocks may be rearranged while remaining within the scope of the present disclosure. Similarly, the accompanying method claims present operations in a sample order, and are not necessarily limited to the specific order presented.

While certain exemplary embodiments have been described above in detail and shown in the accompanying drawings, it is to be understood that such embodiments are merely illustrative of and not restrictive of the broad invention. In particular, it should be recognized that the teachings herein apply to a wide variety of apparatuses and methods. It will thus be recognized that various modifications may be made to the illustrated and other embodiments described above, without departing from the broad inventive scope thereof. In view of the above it will be understood that the invention is not limited to the particular embodiments or arrangements disclosed, but is rather intended to cover any changes, adaptations or modifications which are within the scope of the invention as defined by the appended claims.

## Claims

1. A system for generating an indication of ischemia associated with a heart of a patient, comprising:
delay determining means for determining an electrical depolarization to mechanical contraction delay for a ventricle of a heart;
means for determining whether the delay is more than an expected delay; and
means for generating an indication of ischemia in accordance with a determination that the delay is more than the expected delay.

2. The system of claim 1, wherein the means for determining whether the delay is more than the expected delay is arranged to compare the delay to a baseline delay value.

3. The system of claim 2, wherein the means for determining whether the delay is more than the expected delay is further arranged to adjust the baseline delay value in accordance with an activity level and/or a position of the patient.

4. The system of claim 1, wherein the means for determining whether the delay is more than the expected delay is arranged to determine that electrical depolarization to mechanical contraction delay for the ventricle has increased, or to determine whether there has been an increase in a percentage of a cardiac cycle associated with electrical depolarization to mechanical contraction delay for the ventricle.

5. The system of claim 1, wherein the delay means is arranged to measure a delay between an R-wave and an upslope of a pressure wave for the ventricle, or to measure left atrial pressure.

6. The system of claim 1, wherein the ventricle comprises a right ventricle or a left ventricle.

7. The system of claim 1, wherein generating the indication of ischemia further comprises corroborating an intracardiac electrogram-based indication of ischemia.

8. A method of generating an indication of ischemia associated with a heart of a patient, comprising:
determining an electrical depolarization to mechanical contraction delay for a ventricle of a heart;
determining whether the delay is more than an expected delay; and
generating an indication of ischemia in accordance with a determination that the delay is more than the expected delay.

9. The method of claim 8, wherein determining whether the delay is more than the expected delay comprises comparing the delay to a baseline delay value.

10. The method of claim 9, further comprising adjusting the baseline delay value in accordance with an activity level and/or a position of the patient.

11. The method of claim 8, wherein determining whether the delay is more than the expected delay comprises determining that electrical depolarization to mechanical contraction delay for the ventricle has increased, or determining whether there has been an increase in a percentage of a cardiac cycle associated with electrical depolarization to mechanical contraction delay for the ventricle.

12. The method of claim 8, wherein determining the electrical depolarization to mechanical contraction delay comprises measuring a delay between an R-wave and an upslope of a pressure wave for the ventricle, or measuring left atrial pressure.

13. The method of claim 8 wherein the ventricle comprises a right or a left ventricle.

14. The method of claim 8, wherein generating the indication of ischemia further comprises corroborating an intracardiac electrogram-based indication of ischemia.
